# FASCICULE DE BREVET EUROPEEN

(11) **EP 2 875 117 B1**
(45) Date de publication et mention de la délivrance du brevet: **11.04.2018**
(21) Numéro de dépôt: 13740270.7
(22) Date de dépôt: 22.07.2013
(51) Int. Cl.: C12M 1/26, C12Q 1/24, C12M 1/12

(54) **PROCÉDÉ D'ISOLEMENT DE MICRO-ORGANISMES SUR UN MILIEU DE CULTURE ET DISPOSITIF ASSOCIÉ**
VERFAHREN ZUR ISOLIERUNG VON MIKROORGANISMEN AUF EINEM KULTURMEDIUM UND ZUGEHÖRIGE VORRICHTUNG
METHOD FOR ISOLATING MICROORGANISMS ON A CULTURE MEDIUM AND RELATED DEVICE

(30) Priorité: 20.07.2012 FR 1257047
(43) Date de publication de la demande: 27.05.2015
(73) Titulaire: Biomerieux, 69280 Marcy-l'Étoile (FR)
(72) Inventeur: FLANDROIS, Jean-Pierre, F-69003 Lyon (FR); LIMON, Bernard, F-01250 Rignat (FR); ROZAND, Christine, F-69280 St Genis les Ollières (FR); MONTET, Marie-Pierre, F-69290 Grézieu la Varenne (FR)
(74) Mandataire: Morel, Anne-Aurore
(86) Numéro de dépôt international: PCT/EP2013/065451
(87) Numéro de publication internationale: WO 2014/013089

(56) Documents cités:
- EP-A2- 0 122 581
- WO-A1-99/47637
- WO-A1-2005/071055
- WO-A1-2010/078404
- FR-A1- 2 182 073
- US-A- 2 672 431
- US-A- 4 565 783

## Description

La présente invention concerne, de façon générale, le domaine de l'analyse microbiologique. Plus particulièrement, elle porte sur un procédé d'isolement d'au moins un micro-organisme issu d'un échantillon contaminé.

L'isolement de micro-organismes sur milieu de culture gélifié, à partir d'un échantillon à analyser ou d'une suspension de micro-organismes, est une étape souvent indispensable à de nombreux procédés d'analyse microbiologique. Cette étape est notamment utilisée pour réaliser des identifications, vérifier la pureté microbienne d'un échantillon ou encore effectuer un dénombrement bactérien par comptage des colonies isolées ainsi obtenues.

Les techniques d'isolement ont pour objectif d'obtenir à la surface d'un milieu nutritif gélifié des colonies directement utilisables (CDU) pour identifier et déterminer la sensibilité aux antibiotiques. Elles sont bien connues de l'homme du métier, la technique des stries étant la technique de référence. Cette dernière consiste à déposer l'inoculum par frottement sur une surface avec une probabilité égale par unité de surface parcourue. La densité locale distribuée diminue approximativement de façon exponentielle lors du parcours de l'instrument. Ainsi, plusieurs zones d'étalement à partir d'un seul inoculum sont réalisées, avec ou sans chevauchements des zones, afin d'obtenir l'effet adéquat de distribution et un appauvrissement en bactéries lors du segment d'étalement subséquent. En fin d'étalement les cellules sont suffisamment isolées les unes des autres pour que les développements microbiens sous forme de CDU (colonies visibles ou microcolonies) ne soient pas superposées, même partiellement. Cette technique peut être aussi réalisée par un seul ensemencement continu en spirale au moyen d'une platine tournante ou par une bille magnétique entraînée par un dispositif produisant un ensemencement continu non recouvrant ou éventuellement partiellement chevauchant.

Une autre technique largement répandue consiste en l'isolement sur boîte de milieu gélifié par étalement en surface. Dans ce cas, un mélange de cellules à une concentration cellulaire faible permettant de mettre en culture 30 à 300 cellules est étalé à la surface du gel d'une boîte de Petri de 9 cm de diamètre, chaque cellule se développant en une colonie isolée. Lorsque moins de 30 cellules sont mises en contact avec le gel nutritif des problèmes statistiques faussent l'exactitude du dénombrement.

Lorsque l'effectif dénombré est au-delà de 300 cellules, des erreurs de dénombrement apparaissent du fait du chevauchement des surfaces des colonies. L'étalement est habituellement réalisé par un instrument comportant par exemple une partie linéaire qui est en contact avec le gel ou par emploi de billes de quelques millimètres de diamètre roulant aléatoirement sur la surface par un mouvement désordonné. Cette technique n'est adaptée qu'à un échantillon faiblement contaminé ou dilué car un nombre élevé de cellules augmente la probabilité de confluence des colonies résultant de la croissance.

Il est également possible d'effectuer un isolement sur boite par inoculation en profondeur. L'échantillon de départ est dilué plusieurs fois de manière à réduire suffisamment la population microbienne et obtenir des colonies séparées. De petit volumes de chacun des échantillons dilués sont alors mélangés à un gel liquide, habituellement de la gélose maintenue en surfusion environ à 45°C. Les mélanges sont immédiatement versés dans des boites de culture stériles et après gélification et incubation, chaque cellule est immobilisée et forme une colonie.

Certaines méthodes manuelles se sont vues automatisées grâce à la mise au point de dispositif. C'est le cas par exemple du document EP-0 242 114 qui décrit un appareil et une méthode d'ensemencement d'un milieu de culture avec un échantillon. La méthode consiste à réaliser plusieurs segments d'étalement à partir d'un inoculum. Ces segments sont sous la forme d'arc de cercle et sont réalisés au moyen de quatre têtes d'étalement différentes. Un effet de dilution de l'échantillon est obtenu par chevauchement partiel des segments subséquents. La méthode décrite dans le document est en fait très proche de la méthode d'isolement manuelle de référence.

Plus récemment, de nouvelles méthodes d'isolement ont vu le jour, permettant l'amélioration de l'épuisement bactérien par l'usage d'un applicateur optimisé tel que décrit dans le document WO-A-2005071055. C'est le cas notamment de la méthode d'ensemencement mise en oeuvre dans l'automate commercialisé par la demanderesse sous la référence PREVI™ Isola.

Néanmoins, ces techniques d'isolement ne sont efficaces que sur des milieux de culture gélifiés.

Dans les domaines du diagnostic clinique et du contrôle microbiologique industriel agroalimentaire, pharmaceutique ou cosmétique, les milieux de culture gélifiés en boite de Petri, le plus souvent gélosés constituent depuis la fin du XIXème siècle un outil indispensable à la détection et à l'identification des micro-organismes pathogènes.

Un grand nombre de produits a cependant été développé pour remplacer la boite de Petri. Pour ces produits, la réhydratation se fait in situ, c'est à dire directement dans l'espace servant à l'inoculation et à l'incubation. L'un d'eux, le système petrifilm™, comprenant des nutriments réhydratables, est très largement utilisé. Un autre système développé par la société Nissui Pharmaceutical, le Compact Dry, consiste également en un milieu déshydraté. Le document FR 2 182 073 décrit un dispositif comprenant une couche absorbante à laquelle une membrane micro-poreuse est associée par l'intermédiaire d'une interface composite de la couche et de la membrane. Ce dispositif incorpore un milieu nutritif liquide qui est ensuite désséché. Ces milieux de culture ont pour avantage de se conserver plus longtemps qu'un milieu de culture gélosé prêt à l'emploi. Ils peuvent également, comme le pétrifilm™, présenter un faible encombrement et ainsi utiliser un faible espace d'incubation. La surface disponible pour la culture est limitée et les nutriments disponibles par cellule sont de concentration adaptée pour donner des colonies de diamètre plus petit que sur milieu gélifié classique, la forme des colonies pouvant être aussi modifiée du fait de la faible solidité du gel utilisé ou des coefficients élevés de diffusion des bactéries.

Néanmoins, l'isolement de colonies sur ces milieux n'est possible que par inclusion dans le gel formé lors de la réhydratation, et donc à partir d'un échantillon de départ faiblement contaminé ou ayant subi une série de dilutions. La concentration finale, à déposer sur le milieu, doit être inférieure à 300 cfu/ml (Unité Formant Colonie), ces données classiques dépendant de la taille de la colonie.

De plus, ces milieux ne peuvent subir la contrainte mécanique d'un moyen d'isolement par épuisement sans être détériorés. Ainsi, une des problématiques majeures de ces milieux de culture réhydratables in situ, c'est à dire directement dans l'espace servant à l'inoculation et à l'incubation, est qu'ils ne sont pas compatibles avec l'isolement mécanique manuel ou automatisé de micro-organismes. Ils nécessitent, lorsque l'échantillon de départ est largement contaminé (au-delà de 300 Unités Formant Colonie/ml), la réalisation d'une série de dilutions, impliquant une prise d'essai plus importante, une perte de temps et la consommation d'un nombre important de réactifs (milieu de culture, tubes de diluants, etc.), générateur d'un volume élevé de déchets (autoclavage, coût du traitement). De plus, si un grand nombre de dilutions est réalisé, il existe un risque de perdre, par l'effet de la dilution le micro-organisme pathogène cible, si celui-ci est présent en faible quantité par rapport à la micro flore totale.

Il ressort de l'état de la technique considéré qu'il n'existe pas de méthode d'isolement, simple à mettre en oeuvre à partir d'un échantillon à analyser ou d'une suspension bactérienne, sur un milieu de culture réhydratable in situ qui permette d'obtenir, des colonies isolées.

Un premier objectif de la présente invention est donc de fournir un procédé d'isolement de micro-organismes et un dispositif associé plus performants et plus simples à mettre en oeuvre que les procédés et dispositifs de l'état de la technique.

Un deuxième objectif de la présente invention est de fournir un dispositif et un procédé d'isolement de micro-organismes exploitable sur un milieu de culture réhydratable, ou réhydraté peu avant ou simultanément à l'isolement microbien, directement dans l'espace servant à l'inoculation et à l'incubation.

Un troisième objectif de la présente invention est de fournir un procédé d'isolement de micro-organismes à partir d'un échantillon ayant une concentration microbienne initiale élevée.
Un quatrième objectif de la présente invention est de fournir un procédé d'isolement compatible également avec le dénombrement des micro-organismes.
Un cinquième objectif de la présente invention est de fournir un dispositif et procédé d'isolement compatible avec l'utilisation de milieux chromogéniques.

Ces objectifs parmi d'autres sont atteints par la présente invention qui propose un procédé d'isolement d'au moins un micro-organisme issu d'un échantillon susceptible d'être contaminé par ledit micro-organisme comprenant les étapes suivantes :
(a) fournir un dispositif pour l'isolement de micro-organismes comprenant
   ∘ une couche inférieure imperméable à l'eau
   ∘ une couche nutritive, disposée sur la couche inférieure, comprenant un support contenant un milieu de culture déshydraté, ladite couche nutritive étant obtenue par imprégnation, de préférence à sec, dudit support par le milieu de culture déshydraté, puis calandrage
   ∘ une couche d'isolement perméable aux éléments compris dans la couche nutritive, apte à retenir les bactéries à sa surface et recouvrant tout ou partie de la couche nutritive
   ∘ une couche supérieure protectrice
(b) déposer un volume déterminé de l'échantillon sur la couche d'isolement
(c) isoler les micro-organismes par épuisement ou par nappage de l'échantillon à l'aide un moyen d'isolement,
(d) incuber le dispositif pendant un temps et à une température prédéterminés permettant la croissance des micro-organismes
ledit procédé comprenant également au moins une étape de réhydratation du milieu de culture avec un volume de liquide prédéterminé avant ou simultanément à l'étape b) et/ou c) et/ou d), de préférence avant ou simultanément à l'étape b) et/ou c).

Selon un mode de réalisation préféré, le dispositif mentionné à l'étape a) est obtenu par le procédé comprenant les étapes suivantes :
- verser ledit volume de liquide prédéterminé sur la couche imperméable à l'eau,
- disposer la couche d'isolement sur la couche nutritive, le tout étant placé sur la couche imperméable à l'eau ayant préalablement reçu ledit volume de liquide prédéterminé afin de permettre la réhydratation instantanée et homogène dudit milieu de culture déshydraté, et
- superposer ensuite la couche supérieure protectrice.

En d'autres termes, ledit dispositif est avantageusement obtenu par superposition successive des couches suivantes : 1) couche imperméable à l'eau (ayant de préférence reçu ledit volume de liquide prédéterminé), 2) couche nutritive, 3) couche d'isolement et 4) couche supérieure protectrice, au moins les couches nutritive 2) et d'isolement 3) étant mécaniquement indépendantes l'une de l'autre, de manière à pouvoir être aisément désolidarisées. Ceci offre notamment l'avantage de pouvoir commercialiser et/ou stocker séparément chacune des deux couches susvisées. Ces dernières seront ensuite aisément superposées par l'utilisateur. De préférence, toutes les couches sont mécaniquement indépendantes les unes des autres, de manière à pourvoir être aisément désolidarisées les unes des autres.

Par « couches mécaniquement indépendantes l'une de l'autre » (ou « les unes des autres »), l'on entend des couches non-liées entre elles par au moins un moyen de liaison, ledit moyen de liaison pouvant être notamment de nature :
- physique, comme par exemple une ou plusieurs agrafes, ou
- chimique, tel que la colle, la fusion de tout ou partie des deux couches sous l'action d'un solvant pour donner lieu à une couche composite/hybride (également dénommée « couche liante »), ou encore un gel ou un agent gélifiant s'interposant au moins partiellement entre lesdites couches (à savoir au moins partiellement à l'interface entre lesdites couches).

Outre l'avantage susvisé (commercialisation et/ou stockage séparé des couches nutritive et d'isolement), la Demanderesse a découvert, contre toutes attentes, que le fait de se passer d'un moyen de liaison formant interface entre la couche nutritive et la couche d'isolement permet, en réduisant la distance entre ces deux couches, d'obtenir une meilleure croissance et/ou survie des micro-organismes déposés sur la couche d'isolement. En effet, un tel moyen de liaison est de nature à ralentir le passage des éléments nutritifs de la couche nutritive réhydratée vers les micro-organismes présents sur la couche d'isolement, réduisant ainsi la croissance et/ou les chances de survie de ces micro-organismes.

On entend par échantillon une petite partie ou petite quantité séparée d'une entité par un acte soustractif dénommé habituellement prélèvement, à des fins d'analyse.

L'échantillon peut être d'origine biologique, humaine, animale, végétale ou environnementale. Il peut concerner un produit au cours d'un processus industriel ou un produit fini, par exemple alimentaire. Il peut donc correspondre à un prélèvement de fluide biologique (sang total, sérum, plasma, urine, liquide céphalo-rachidien, sécrétion organique), un prélèvement tissulaire ou des cellules isolées. Il peut être d'origine industrielle, soit, selon une liste non exhaustive un prélèvement d'air, un prélèvement d'eau, un prélèvement effectué sur une surface, une pièce ou un produit en cours de traitement ou manufacturé, un produit d'origine alimentaire. Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc.) et les produits constitutifs ou annexes du produit fini. Un échantillon alimentaire peut enfin être issu d'une alimentation destinée aux animaux, telle que notamment des farines animales. Ce prélèvement peut subir préalablement à son analyse une préparation de type enrichissement, extraction, concentration, purification, selon des méthodes connues de l'homme du métier. Selon un mode de réalisation préférentiel, le volume d'échantillon déposé sur le milieu de culture est compris entre 10 et 1000µl.

Au sens de la présente invention, le terme micro-organisme recouvre les bactéries à Gram positif ou gram négatif, les levures, les moisissures, les amibes et plus généralement, les organismes unicellulaires, invisibles à l'oeil nu, qui peuvent être manipulés et multipliés en laboratoire.

Selon un mode préféré de réalisation de l'invention, le micro-organisme est une bactérie, gram négative ou positive, ou une levure.

A titre de bactéries Gram positives, on peut citer les bactéries des genres suivants : *Enterococcus, Streptococcus, Lactobacillus, Bifidobacterium, Staphylococcus, Bacillus, Listeria, Clostridium, Mycobacteria, Nocardia*, *Corynebacteria, Micrococcus et Deinococcus.*

A titre de levures, on peut citer les levures des genres suivants : *Candida, Cryptococcus, Saccharomyces et Trichosporon.*

A titre de moisissures, on peut citer les moisissures des genres suivants : *Aspergillus, Penicillium, Cladosporium.*

La présente invention concerne également un dispositif pour la culture de micro-organismes comprenant
∘ une couche inférieure imperméable à l'eau
∘ une couche nutritive, disposée sur la couche inférieure, comprenant un support contenant un milieu de culture déshydraté, ladite couche nutritive étant obtenue par imprégnation, de préférence à sec, dudit support par le milieu de culture déshydraté, puis calandrage
∘ une couche d'isolement perméable aux éléments compris dans la couche nutritive, apte à retenir les bactéries à sa surface et recouvrant tout ou partie de la couche nutritive
∘ une couche supérieure protectrice.

De préférence, la couche nutritive et la couche d'isolement sont mécaniquement indépendantes l'une de l'autre, tel qu'indiqué précédemment.

Au sens de la présente invention, la couche nutritive comprend un support contenant un milieu de culture déshydraté. Le support peut être à base de divers composés absorbants à très fort pouvoir de rétention d'eau tels que la rayonne, le coton, les fibres cellulosiques naturelles ou modifiées chimiquement comme la carboxy-méthyl cellulose, les polymères chimiques absorbants ou super-absorbants tels que sels de polyacrylate, copolymère acrylate/acrylamide. Ce support peut être imprégné d'un milieu de culture sous forme liquide puis déshydraté, c'est à dire ayant une Aw (Activity of water, Activité en eau) incompatible avec le développement microbien. Alternativement, il a été recouvert ou imprégné à sec d'un milieu de culture ou de ses constituants sous forme de poudre. Alternativement, l'imprégnation liquide peut, après déshydratation être complétée par ajout de poudre selon les moyens décrits précédemment.

On entend par milieu de culture, un milieu comprenant tous les éléments nécessaires à la survie et/ou à la croissance de micro-organismes. En pratique, l'homme du métier choisira le milieu de culture en fonction des micro-organismes cibles, selon des critères parfaitement connus et à la portée de cet homme de l'art.

La couche nutritive selon l'invention peut contenir d'éventuels additifs comme par exemple : des peptones, un ou plusieurs facteurs de croissance, des hydrates de carbone, un ou plusieurs agents sélectifs, des tampons, colorants, un ou plusieurs gélifiants, des hydrogels, agent visqueux, etc..

Préférentiellement, le milieu ne contient pas ou peu d'agents gélifiants à froid, tels que, par exemple, agar, agarose, poloxamères, gomme guar, xanthane... D'une manière générale, la couche nutritive peut en sus contenir un substrat permettant la détection d'une activité enzymatique ou métabolique des micro-organismes cibles grâce à un signal détectable directement ou indirectement. Pour une détection directe, ce substrat peut être lié à une partie faisant office de marqueur, fluorescent ou chromogène. Pour une détection indirecte, la couche nutritive selon l'invention peut comporter en sus un indicateur de pH, sensible à la variation de pH induite par la consommation du substrat et révélant la croissance des micro-organismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'hydroxycoumarine ou les dérivés de la résorufine. Ainsi, le substrat fluorescent de PC-PLC préférentiellement utilisé pour la mise en oeuvre du procédé selon l'invention correspond au 4-Méthyl-Umbelliferyl-Choline Phosphate (4 MU-CP).

Selon l'invention, après imprégnation à sec de la couche nutritive par le milieu de culture déshydraté, celle-ci fait l'objet d'une opération de calandrage. Le calandrage, par la pression et la température de chauffe générées, permet la rétention et le maintien stable dans le temps du milieu de culture déshydraté dans la couche nutritive, en assurant la rétention des éléments nutritifs dans la couche nutritive. Il permet également l'obtention d'une surface rigoureusement lisse et plane de la couche nutritive. Ceci est particulièrement avantageux pour assurer une bonne cohésion entre la couche nutritive et la couche d'isolement lorsque cette dernière est disposée sur la couche nutritive. Le calandrage permet, outre l'accélération de la réhydratation de la couche nutritive par rapport à une couche nutritive non-calandrée, une compression des fibres constituant la couche nutritive. Cette compression, associée à la présence du milieu déshydraté au sein de la couche nutritive, génère une forte augmentation du pouvoir capillaire de cette dernière, provoquant sa réhydratation quasi-instantanée et générant un phénomène d'aspiration de la couche d'isolement disposée à sa surface. La couche d'isolement se retrouve dès lors plaquée contre la couche nutritive (tout en étant mécaniquement indépendante de cette dernière), assurant ainsi l'absence d'espace entre les deux couches et en conséquence une croissance et/ou survie microbienne optimale(s) sur toute la surface de la couche d'isolement. Il n'y a dès lors pas besoin de recourir à un moyen de liaison (par exemple une couche liante) entre la couche d'isolement et la couche nutritive. Ceci représente un avantage significatif, dans la mesure où un tel moyen de liaison ralentit le passage des éléments nutritifs de la couche nutritive réhydratée vers les micro-organismes présents sur la couche d'isolement, réduisant ainsi la croissance et/ou les chances de survie de ces micro-organismes.
Tel qu'indiqué précédemment, la possibilité de désolidariser la couche nutritive et la couche d'isolement, permettant ainsi de commercialiser et/ou stocker les deux couches séparément, représente un réel plus industriel. Un tel effet technique permet dès lors de considérer les couches nutritives et d'isolement indépendamment l'une de l'autre, y compris d'un point de vue commercial et/ou logistique (stockage). Ceci représente un avantage significatif pour l'utilisateur qui dispose notamment de la possibilité de combiner à dessein des couches nutritives et d'isolement avec des propriétés différentes et adaptées aux micro-organismes à mettre en évidence.

On entend par couche d'isolement une couche dont le principal constituant est un matériau qui par sa nature, sa taille, son arrangement stérique, retient les micro-organismes à sa surface tout en étant perméable aux éléments compris dans la couche nutritive située sous la couche d'isolement.

La couche d'isolement peut être à base de un ou plusieurs matériaux ou dérivé de ces matériaux tels que le latex, le polytetrafluoroethylene, le poly(vinylidene) fluoride, le polycarbonate, le polystyrène, le polyacide, le polysulphone, le polyethersulfone, la cellulose, un mélange de celluloses et nitrocellulose. Avantageusement, la couche d'isolement est poreuse, préférentiellement il s'agit d'une membrane poreuse perméable aux éléments compris dans la couche nutritive, apte à retenir les micro-organismes à sa surface et recouvrant tout ou partie de la couche nutritive. La Demanderesse a découvert que les membranes de microfiltration de l'eau (et d'une manière générale des liquides) actuellement commercialisées présentent généralement les propriétés requises pour être utilisées comme couche d'isolement. Elles permettent d'obtenir une très bonne résistance au déchirement lors de la manipulation, une porosité maîtrisée, un caractère parfaitement lisse, une fine épaisseur et la plupart du temps une forte hydrophilie. Leur couleur, généralement blanche, permet d'optimiser la différentiation de colonies colorées sur leur surface. La Demanderesse a découvert, de manière surprenante, que l'utilisation de ces membranes de filtration non pas pour une action de filtration « traditionnelle » de liquides (comme on le fait pour l'eau ou d'une manière générale pour des liquides) mais comme support lisse et résistant pour effectuer l'isolement de l'échantillon à tester, sur leur surface était particulièrement adaptée à la mise en oeuvre de la présente invention. La capacité de filtration et l'hydrophilie de la membrane de filtration, quant à elles, sont mises à profit pour permettre et optimiser le passage des liquides nutritifs présents dans la couche nutritive (après sa réhydratation) vers la couche d'isolement tout en empêchant la migration en sens inverse des bactéries, levures et moisissures ensemencés à la surface de la couche d'isolement.

Aux fins de la présente demande, les susdites membranes de filtration sont indifféremment désignées « membranes de filtration », « membranes de microfiltration » ou encore « membranes filtrantes », ces expressions étant synonymes les unes des autres. Ces membranes de filtration sont comprises dans le groupe constitué par les membranes poreuses.

Dans un mode de réalisation de l'invention, la couche d'isolement est active. Elle peut ainsi contenir des sites spécifiques récepteurs des bactéries tels que anticorps ou fragments d'anticorps, bactériophages ou fragments de bactériophages, aptamères ou tous ligands spécifiques qui pourront être fixés de façon covalente ou indirectement par des éléments biochimiques affines dont l'un au moins est fixé de façon covalente comme par exemple un système utilisant de la streptavidine et de la biotine.

Alternativement, la couche d'isolement est active en contenant des composés inhibiteurs ou destructeurs des bactéries tels que bactériophages, peptides anti-bactériens, antibiotiques qui pourront être fixés ou non dans ou sur la couche d'isolement. Les composés peuvent être fixés de façon covalente ou indirectement par des éléments biochimiques affines. Encore, dans un autre mode de réalisation de l'invention, la couche d'isolement est active en contenant des éléments stimulants ou accélérant la croissance des micro-organismes. A titre d'exemple, un agent visqueux tels que le Polyéthylène glycol est disposé sur la couche d'isolement.

L'isolement peut être effectué sur tout ou partie de la couche d'isolement, définissant la surface utile pour l'isolement.

La couche d'isolement est constituée d'une surface permettant le passage des éléments du milieu nutritif et des agents sélectifs ou réactifs.

Avantageusement, la couche d'isolement comporte des pores dont le diamètre est compris entre 0,01 et 0,8µm préférentiellement entre 0,2 µm et 0,6 µm de manière à retenir les bactéries, levures et moisissures sur sa surface. Selon un mode de réalisation particulier, la couche d'isolement comporte des pores dont le diamètre est compris entre 0,25 µm et 0,6 µm, par exemple entre 0,3 µm et 0,6 µm, ou encore entre 0,4 µm et 0,6 µm.

Alternativement, il peut s'agir d'une couche ne présentant pas de pores mesurables comme une membrane de dialyse, une membrane de cellulose perméable à l'eau et aux composés chimiques et définie par sa capacité de rétention des composés d'une taille moléculaire supérieure à un seuil. Avantageusement la membrane permettra de retenir les molécules chimiques d'un poids moléculaire supérieur à 500 000 daltons (ou compris entre 5000 et 500 000 daltons).

De façon avantageuse, la couche d'isolement comprend des zones confinées qui permettent de faciliter l'isolement des micro-organismes. Elles peuvent également permettre de diminuer le temps de croissance et donc d'obtenir le résultat plus rapidement.

Dans un mode de réalisation, la couche d'isolement possède des motifs hydrophobes et/ou hydrophiles délimitant spatialement la croissance des bactéries. La taille du motif hydrophile est comprise entre 10 et 500 µm, préférentiellement entre 300 et 500 µm à savoir un diamètre supérieur au pouvoir séparateur de l'oeil. Lorsqu'il s'agit de la microbiologie classique et donc d'une observation visuelle.

Dans un autre mode de réalisation, la couche d'isolement contient des motifs nanostructurés, incompatibles avec la croissance microbienne, délimitant des zones confinées permettant la croissance du micro-organisme.

Les motifs hydrophobes sont agencés sous forme d'un motif de surfaces qui est un quadrillage régulier ou irrégulier, un assemblage d'hexagones réguliers ou irréguliers ou selon un mélange des deux patrons, ou éventuellement d'autres formes.

Les matériaux souhaitables pour la réalisation de ce motif hydrophobe incluent la cire, de l'encre hydrophobe, les résines époxy, huile silicone, cire silicone, résine polystyrène, oxyde d'alumine, polyfluorothylène, etc.

La couche d'isolement recouvre tout ou partie de la couche nutritive. La couche d'isolement est positionnée sur la couche nutritive, la recouvrant en partie afin de permettre la réhydratation du milieu par un liquide. Dans un mode de réalisation préféré, la couche nutritive peut être recouverte entièrement par la couche d'isolement. Le dispositif peut contenir au moins un moyen de réhydratation en contact avec la couche nutritive tel qu'un réservoir annexé au dispositif ou inclus dans ce dernier et/ou des canaux permettant la réhydratation de la partie inférieure ou latérale de la couche nutritive, de préférence de la partie inférieure de cette dernière.

La surface utile pour l'isolement peut être entourée d'une zone ne permettant pas le développement microbien. Il peut s'agir d'une zone faite d'un matériau imperméable ou d'une zone hydrophobe. Avantageusement, ladite zone peut être structurée pour guider l'utilisation manuelle.

On entend par moyen d'isolement, un moyen permettant la mise en contact des micro-organismes avec le milieu de culture en se déplaçant sur ce dernier afin de déposer l'échantillon. L'échantillon s'appauvrissant au fur et à mesure du frottement de l'échantillon et/ou du moyen d'isolement sur la couche d'isolement, des cellules isolées sont déposées permettant d'obtenir, après croissance, des colonies isolées.

Le moyen d'isolement présente une ou plusieurs surface(s) de contact avec ledit milieu de culture. Un tel moyen peut être utilisé manuellement, par exemple une oese, une anse de platine une pipette, une tige rodée, une tige comportant une boule terminale, un écouvillon. Il peut également s'agir de billes.

Avantageusement, le procédé selon l'invention peut être mis en oeuvre au moyen d'un système automatisé. Un système particulièrement adapté est le système PREVI™ Isola tel que protégé dans la demande de brevet WO-A-2005071055 et commercialisé par la demanderesse.

Selon l'invention, le milieu de culture est réhydraté avec un volume de liquide prédéterminé.

Un volume approprié de liquide se propage dans le milieu de culture. En pratique, l'homme du métier choisira le volume approprié de liquide en fonction de la viscosité du liquide, le diamètre de la couche nutritive, ceci afin de réhydrater le milieu et permettre la croissance des micro-organismes. L'étape de réhydratation a lieu avant ou simultanément à l'étape b) et/ou c) et/ou d), de préférence avant ou simultanément à l'étape b) et/ou c).Dans un mode de réalisation particulier, le liquide est ajouté manuellement à l'aide d'une pipette ou automatiquement. Dans un autre mode, le liquide est contenu dans au moins un réservoir intégré au dispositif et/ou des canaux permettant la réhydratation de la couche nutritive. Le liquide se répand alors dans la couche nutritive par simple pression du réservoir. Un avantage d'une réhydratation par la partie inférieure et/ou latérale, de préférence par la partie inférieure, est qu'elle permet une hydratation homogène de toute la couche nutritive. Ce mode de réalisation évite notamment aux nutriments de la couche nutritive d'être entrainés par le liquide dans la partie inférieure du dispositif.

Selon un mode de réalisation de l'invention, le liquide utilisé pour reconstituer le milieu de culture est une solution aqueuse. Selon un mode de réalisation particulier, le liquide contient des microparticules avec des enveloppes lipidiques, préférentiellement, le liquide contient des globules rouges.

Le liquide peut également être un tampon ou une solution contenant des compléments du milieu de culture, tels que des antibiotiques ou des substrats.

Selon l'invention, le dispositif comprend de part et d'autre de la couche nutritive et la couche d'isolement
une couche inférieure imperméable à l'eau
une couche supérieure protectrice.

La couche supérieure peut être translucide ou transparente permettant la visibilité des colonies au travers de cette couche. Préférentiellement, la couche supérieure protectrice est séparée des colonies par un moyen de séparation. Le moyen de séparation peut être une paroi latérale ou tout autre moyen permettant à la couche supérieure de ne pas être en contact avec les colonies.

Avantageusement, la couche supérieure pourra reposer sur les parties de la couche d'isolement ne permettant pas le développement microbien comme les zones périphériques ou les motifs hydrophobes.

La couche supérieure peut être attachée par un des côtés à une des autres couches du dispositif par n'importe quel moyen à savoir, par exemple, un moyen adhésif ou mécanique.

Elle permet également d'éviter les contaminations au cours de l'incubation. Elle est imperméable aux bactéries et limite la perte de vapeur d'eau. En effet, le dispositif est incubé pendant un temps et à une température prédéterminés permettant la croissance des micro-organismes indépendamment des conditions d'humidité ambiante. Ainsi, la nature de la couche supérieure est choisie de façon à permettre les échanges gazeux nécessaires à la croissance des micro-organismes tout en permettant une hydratation locale. La couche inférieure est imperméable à l'eau. De préférence, cette couche inférieure est rigide, permettant une meilleure prise en main du dispositif Elle est fabriquée à partir de composés tels que le polyester, polypropylènes, polystyrène. De préférence, elle est fabriquée à partir de cellulose. Il peut s'agir de carton ou de papier associé à un film imperméable à l'eau. Elle peut contenir des canaux thermoformés qui serviront à la bonne réhydratation de la couche nutritive.

De façon avantageuse, les différentes couches du dispositif sont fabriquées à partir de matériaux recyclables.

Selon un mode de réalisation particulier de l'invention, la couche inférieure et/ou la couche nutritive et/ou la couche d'isolement est translucide ou transparente.

Selon un mode de réalisation particulier de l'invention, le dispositif comprend également un code d'identification tel que les codes-barres ou les étiquettes RFID.

Un dispositif selon l'invention peut être de forme classique, à savoir de forme ronde. Il peut néanmoins être de forme différente et notamment de forme carrée ou rectangulaire. Selon une autre variante, les différentes couches peuvent être de formes différentes. Ainsi, par exemple, les couches supérieure, inférieure et nutritive peuvent être de forme carrée et la couche d'isolement et/ou la surface utile pour l'isolement de forme ronde. Les différentes couches peuvent être de couleurs différentes favorisant la discrimination des bactéries suspectées.

L'invention concerne également l'utilisation d'un dispositif selon l'invention.

L'invention, sa fonctionnalité, ses applications ainsi que ses avantages seront mieux appréhendés à la lecture de la présente description, faite en référence aux figures, dans lesquelles:

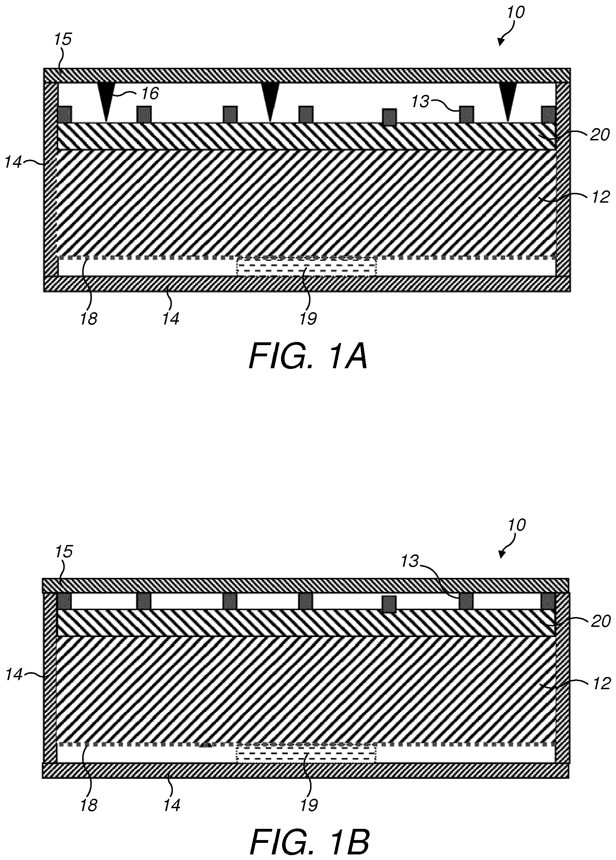

- les figures 1A et 1B sont des représentations schématiques du dispositif selon l'invention. Le dispositif 10 comprend une couche inférieure imperméable à l'eau 14, une couche nutritive 12, disposée sur la couche inférieure, comprenant un milieu de culture déshydraté, une couche d'isolement 20 perméable aux éléments compris dans la couche nutritive, apte à retenir les bactéries à sa surface et recouvrant tout ou partie de la couche nutritive, une couche supérieure protectrice 15. Ce dispositif comprend également des zones hydrophiles/hydrophobes 13, un réservoir 19 et des canaux 18 permettant la réhydratation de la partie inférieure ou latérale de la couche nutritive.
   Le dispositif peut avoir des moyens de séparation 16 permettant à la couche supérieure 15 de ne pas être en contact avec les colonies.
- la figure 2A est une photographie du dispositif selon l'invention dont la surface d'isolement utile est de 25 cm² ;
- la figure 2B représente un agrandissement de la partie centrale du dispositif Le dispositif a été ensemencé par un échantillon d'*Escherichia coli* à une concentration de 10⁸ UFC/ml.
- la figure 3 montre le contenu d'une boîte de Petri ensemencée avec une souche de *Klebsiella pneumoniae* en présence d'un milieu de culture UriSelect™ 4 avec une couche d'isolement consistant en une membrane filtrante en nitrate de cellulose Macherey Nagel, à titre d'exemple;
- la figure 4 montre le contenu d'une boîte de Petri ensemencée avec une souche de *Klebsiella pneumoniae* en présence d'un milieu de culture UriSelect™ 4 avec une couche d'isolement consistant en une membrane filtrante en nitrate de cellulose Sartorius, à titre d'exemple ;
- la figure 5 montre le contenu d'une boîte de Petri ensemencée avec une souche de *Klebsiella pneumoniae* en présence d'un milieu de culture UriSelect™ 4 avec une couche d'isolement consistant en une membrane filtrante en acétate de cellulose, à titre d'exemple ;
- la figure 6 montre le contenu d'une boîte de Petri ensemencée avec une souche de *Klebsiella pneumoniae* en présence d'un milieu de culture UriSelect™ 4 avec une couche d'isolement consistant en une membrane filtrante en polyester;
- la figure 7 montre le contenu d'une boîte de Petri ensemencée avec une souche d'*Escherichia coli* en présence d'un milieu de culture UriSelect™ 4 avec une couche d'isolement consistant en une membrane filtrante en nitrate de cellulose Macherey Nagel, à titre d'exemple;
- la figure 8 montre le contenu d'une boîte de Petri ensemencée avec une souche d'*Escherichia coli* en présence d'un milieu de culture UriSelect™ 4 avec une couche d'isolement consistant en une membrane filtrante en polyester, à titre d'exemple ;
- la figure 9 montre le contenu d'une boîte de Petri ensemencée avec une souche d'*Escherichia coli* en présence d'un milieu de culture UriSelect™ 4 avec une couche d'isolement consistant en une membrane filtrante en acétate de cellulose, à titre d'exemple ;
- la figure 10 montre le contenu d'une boîte de Petri ensemencée avec une souche d'*Escherichia coli* en présence d'un milieu de culture UriSelect™ 4 avec une couche d'isolement consistant en une membrane filtrante en polyester, à titre d'exemple ;
- la figure 11 montre les contenus de quatre boîtes de Petri en présence d'un milieu de culture gélosé UriSelect™ 4 et d'un milieu de culture imprégné UriSelect™ 4 déshydraté sur une couche nutritive comprenant un support non tissé de type Glatfelter, Airlaid 150g/ m² en présence d'un échantillon d'une souche d'*Escherichia coli,* à titre d'exemple;
- la figure 12 montre les contenus de quatre boîtes de Petri en présence d'un milieu de culture gélosé UriSelect™ 4, d'un milieu de culture UriSelect™ 4 déshydraté imprégné dans une couche nutritive comprenant un support non tissé de type Glatfelter, Airlaid 150g/ m² en présence d'un échantillon d'une souche d'*Enterobacter cloacae,* à titre d'exemple ;
- la figure 13 montre les détails de la figure 16, représentant deux contenus de deux boîtes de Petri, à titre d'exemple ;
- la figure 14 montre les contenus de quatre boîtes de Petri en présence d'un milieu de culture UriSelect™ 4 imprégné dans une couche nutritive comprenant un support non tissé de type Glatfelter, Airlaid 150g/ m² en présence d'une couche d'isolement telle qu'une membrane filtrante en acétate de cellulose et en présence d'un échantillon d'une souche de *Clostridium Freundii,* à titre d'exemple ;
- la figure 15 montre le contenu d'une boîte de Petri comprenant une couche nutritive contenant un support non tissé imprégné avec un milieu de culture gélosé de type Chrom ID CPS ID3 en présence d'un échantillon *d'Enterococcus faecalis*, à titre d'exemple ;
- la figure 16 montre le contenu d'une boîte de Petri comprenant une couche nutritive contenant un support non tissé de type Airlaid MH 100 137 imprégné d'un milieu de culture de type Chrom ID CPS3 déshydraté et sans agar ;
- la figure 17 montre le contenu d'une boîte de Petri en présence d'un milieu de culture de type milieu gélosé Chrom ID CPS ID3 et d'une couche nutritive comprenant un support non tissé imprégné d'un milieu de type ChromID CPS3 déshydraté et sans agar en présence d'une couche d'isolement telle qu'une membrane filtrante en polyester et d'un échantillon d'une souche *d'Enterobacter cloacae*, à titre d'exemple;
- la figure 18 montre en détails une couche nutritive comprenant un support non tissé de type Airlaid MH 100 137 imprégné d'un milieu de culture de type Chrom ID CPS ID3 sans agar et en présence d'une couche d'isolement telle qu'une membrane en polyester ;
- la figure 19 montre un autre exemple de support selon la figure 18.

### EXEMPLES

Exemple 1 : Obtention de colonies isolées à partir d'une solution fortement contaminée

### sur un milieu réhydraté Petrifilm (exemple illustratif).

A partir d'une solution calibrée à une charge bactérienne théorique de 10⁸ UFC/ml, 1000 µl de solutions chargées en *Escherichia coli* à différentes concentrations obtenues par dilutions successives d'un facteur 10 sont déposés au centre du film inférieur du Petrifilm®. Le film supérieur du Petrifilm® est rabaissé sur l'échantillon. Un diffuseur plastique, la face concave vers le bas est placé au centre du test Petrifilm®. L'échantillon est uniformément réparti en exerçant une légère pression au centre du diffuseur en plastique. L'inoculum est ainsi réparti sur la totalité de la zone de croissance avant que le gel ne se forme.

**Tableau 1 :**

| Dilution UFC/ml | 10⁸ | 10⁷ | 10⁶ | 10⁵ | 10⁴ | 10³ | 10² | 10 |
|---|---|---|---|---|---|---|---|---|
| Colonies | ≥300 | ≥300 | ≥300 | ≥300 | ≥300 | ≥300 | 78 | 7 |
| | Non comptable | Non comptable | Non comptable | Non comptable | Non comptable | Non comptable | | |

Les résultats montrent que six dilutions sont nécessaires afin d'obtenir des colonies isolées et exploitables.

### Exemple 2 : Obtention de colonies isolées à partir d'une solution fortement contaminée sur un milieu réhydraté (exemple illustratif)

A partir de la même solution calibrée à une charge bactérienne théorique de 10⁸ UFC/ml ayant servie à l'inoculation du Petrifilm®, un ensemencement mécanique par la méthode des cadrans est réalisé sur le dispositif permettant d'obtenir sur une surface limitée (25 cm²) du dispositif des colonies isolées.

Ainsi, le dispositif a été ensemencé par 10 µl (contenu d'une oese) d'une solution calibrée à une charge bactérienne théorique de 10⁸ UFC/ml chargée en *Escherichia coli* et déposé sur le 1^{er} cadran 21 de la surface d'isolement du dispositif dont la surface d'isolement utile est de 25 cm². Le deuxième cadran 22 est ensemencé avec une nouvelle oese en étirant plusieurs stries à partir du cadran 21. Le troisième cadran 23 est ensemencé comme le second sans changer d'oese. Le 4^{ème} cadran 24 est ensemencé par des stries non étirées à partir du cadran 22.

Le dispositif est formé par une couche d'isolement avec des motifs hydrophobes/hydrophiles 28 sur laquelle est réalisé l'isolement. Les motifs hydrophobes/hydrophiles permettent d'améliorer l'isolement, notamment sur une petite surface (25 cm²) en délimitant spatialement la croissance des micro-organismes.
Une couche contenant le milieu de culture réhydraté 25. Une couche inférieure imperméable à l'eau 26 et une couche supérieure translucide venant fermer le dispositif 27.

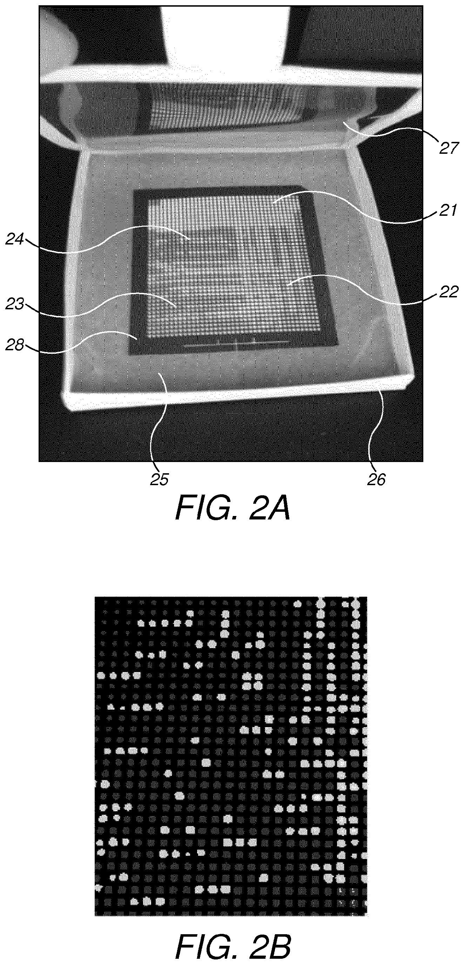

Les figures 2a et 2b montrent que l'isolement mécanique sur le dispositif permet la formation de colonies isolées. L'isolement est ainsi possible grâce à l'utilisation d'un seul dispositif sans dilution préalable.

### Exemple 3: Obtention de colonies isolées sur une couche d'isolement de type membrane filtrante, disposée sur une couche nutritive constituée par un support non tissé imprégné par un milieu nutritif déshydraté.

L'objectif de cet exemple est de comparer les morphotypes et le temps de croissance de colonies se développant sur une membrane poreuse et/ou filtrante (de préférence filtrante) positionnée sur un milieu de culture gélosé ou sur une couche nutritive imprégnée de milieu de culture déshydraté.
La taille et la couleur des colonies issues de différentes espèces bactériennes ensemencées sur ces membranes poreuses et/ou filtrantes sont évaluées par l'opérateur.

### 3.1 Matériel

Les expérimentations décrites ci-dessous concernent notamment des souches d'*Escherichia coli,* de *Clostridium freundii, d'Enterococcus faecalis,* de *Klebsiella pneumoniae, et* d'*Enterobacter cloacae.*

Les couches d'isolement testées pour le présent exemple comprennent :
- une membrane filtrante en polyester (Macherey Nagel Polyester) comprenant des pores de diamètre 0,2 µm, 0,4 µm, 1 µm et 5 µm (référence commerciale : PORAFIL® PE),
- une membrane filtrante en nitrate de cellulose (Macherey Nagel Polyester) comprenant des pores de diamètre 0,2 µm, 0,4 µm, 1 µm et 5 µm (référence commerciale : PORAFIL® NC),
- une membrane filtrante en acétate de cellulose (Macherey Nagel Polyester) comprenant des pores de diamètre 0,2 µm, 0,4 µm, 1 µm et 5 µm (référence commerciale : PORAFIL® CA),
- une membrane filtrante en esters mixtes de cellulose (« cellulose mixed esters » en langue anglaise ; Macherey Nagel Polyester) comprenant des pores de diamètre 0,2 µm, 0,4 µm, 1 µm et 5 µm (référence commerciale : PORAFIL® CM),
- une membrane filtrante en nitrate de cellulose (Sartorius stedim Biotech) comprenant des pores de diamètre 0,45 µm.

Aux fins des présentes expérimentations l'on utilise les supports non tissés suivants :
- Glatfelter, Airlaid 100g/m²,
- Glatfelter, Airlaid concert 150g/m²,
- des supports PDI 60g/m².

Les milieux de culture utilisés pour imprégner le support non tissé dans les présentes expérimentations sont : un bouillon Trypcase soja (TSB-D), un milieu de culure de type UriSelect® 4 (référence commerciale : Bio Rad), ou un milieu de culture du type Chrom ID CPS 3 sans agar.

Les milieux de culture gélosés utilisés au cours des présentes expérimentations sont les suivants : UriSelect®4 (référence commerciale Bio Rad) et Chrom ID CPS 3.

### 3.2 Protocole expérimental

Dans un premier temps, les différentes membranes filtrantes sont testées sur un milieu gélosé de type UriSelect® 4 (cf section 3.3.1 infra).

Dans un second temps, le milieu de culture gélosé UriSelect® 4 est remplacé par les différents supports non tissés imprégnés d'un milieu de culture précédemment cité (cf. section 3.3.2 infra).

Les supports non tissés et imprégnés du milieu de culture sont réhydratés au moyen d'un volume d'eau stérile prédéterminé et d'un inoculum bactérien au moment de réaliser l'analyse. Le volume/la quantité d'eau stérile nécessaire à la réhydratation du support non tissé et imprégné du milieu de culture varie en fonction de la nature du support non tissé et de la dimension de ce celui-ci. Cette donnée est aisément déterminable par l'homme du métier au moyen de ses connaissances générales et, au besoin, de tests de routine.

L'incubation de l'ensemble membrane filtrante et support non tissé imprégné ou membrane filtrante et milieu gélosé s'effectue à une température de 37°C. La lecture visuelle des résultats permettant la détermination du morphotype des colonies et qualité de l'isolement à la surface de la membrane poreuse et/ou filtrante est réalisée dans un premier temps après une durée d'incubation de 24h puis dans un deuxième temps après une durée totale d'incubation de 48h.

### 3.3 Résultats

### 3.3.1 Isolement de différents micro-organismes sur différents types de membranes filtrantes, en présence d'un milieu de culture gélosé.

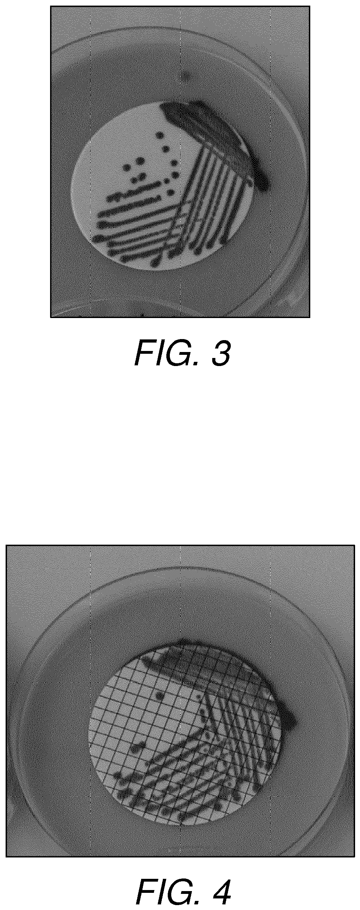

Les figures 3, 4, 5 et 6 montrent l'isolement d'une souche de *Klebsiella pneumoniae* en présence d'un milieu de culture gélosé UriSelect™ 4, après une durée d'incubation de 24h.

Plus précisément, la membrane filtrante de la figure 3 est en nitrate de cellulose (Macherey Nagel).

La membrane filtrante de la figure 4 est en nitrate de cellulose Sartorius (Sartorius).

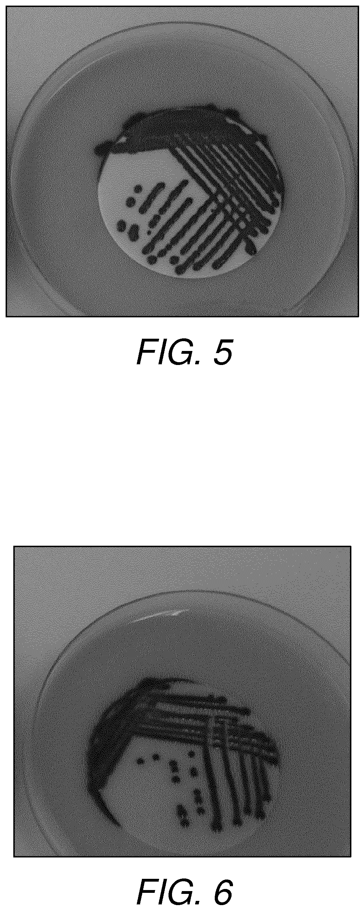

La membrane filtrante de la figure 5 est en acétate de cellulose.

La membrane filtrante de la figure 6 est en polyester.

Sur ces figures 3, 4, 5 et 6, l'on note la présence de colonies directement utilisables (CDU) bien individualisées.

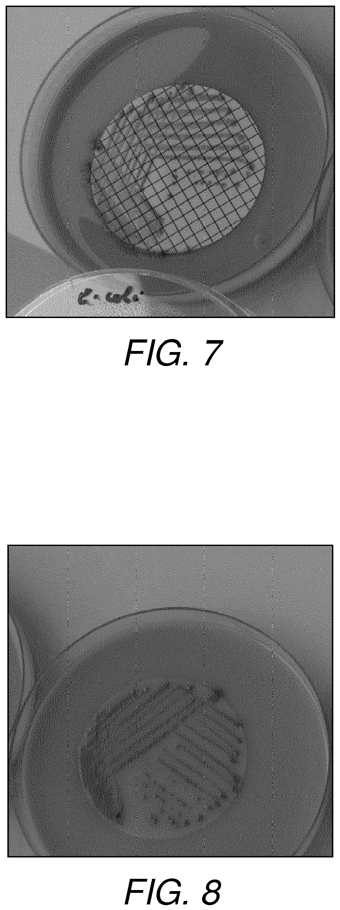

Les figures 7, 8, 9 et 10 montrent la présence d'*Escherichia coli* en présence d'un milieu de culture gélosé UriSelect™ 4, après une durée d'incubation de 24h.

Plus précisément, la membrane filtrante de la figure 7 est en nitrate de cellulose Macherey Nagel.

La membrane filtrante de la figure 8 est en polyester.

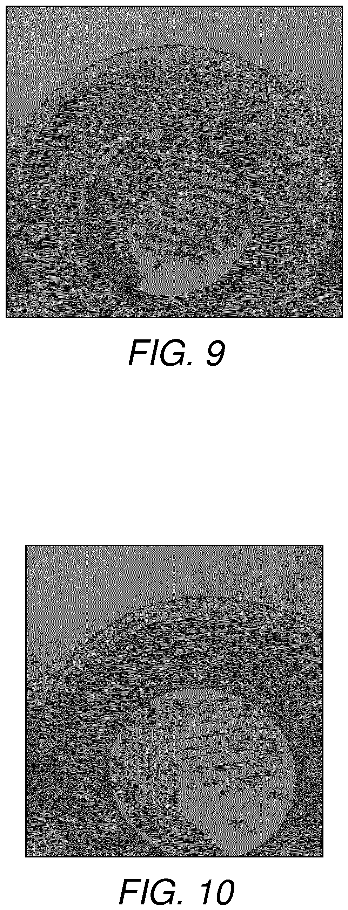

La membrane filtrante de la figure 9 est en acétate de cellulose.

La membrane filtrante de la figure 10 est en polyester.

Sur ces figures 7, 8, 9 et 10, l'on note la présence de colonies directement utilisables (CDU) bien individualisées. La croissance bactérienne est optimale, les morphotypes et la croissance des colonies obtenues sont conformes à ce que l'on attend d'une croissance microbienne directement sur milieu gélosé. L'isolement réalisé sur une membrane poreuse et/ou filtrante aboutit à des morphotypes de colonies et une qualité d'isolement classiquement obtenus avec un isolement réalisé directement sur milieu gélosé.

### 3.3.2 Isolement et énumération/dénombrement de micro-organismes sur des membranes fltrantes déposées sur des supports non tissés imprégnés d'un milieu de culture déshydraté.

La présente expérimentation met en présence un support non tissé imprégné d'un milieu de culture déshydraté. Lors de la réalisation de l'analyse, le support non tissé est imprégné d'eau afin de réhydrater le milieu de culture.

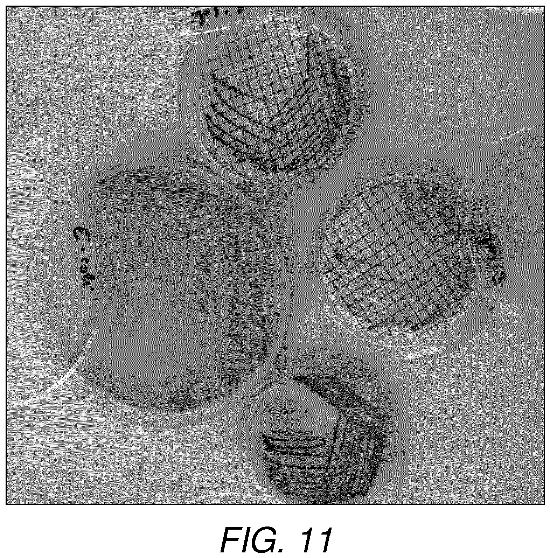

Comme montré sur la figure 11, le support d'imprégnation utilisé est de type Glatfelter, Airlaid 150g/ m². Ce dernier est imprégné avec du milieu de culture UriSelect™ 4 déshydraté.

Les contenus des boîtes de Petri montrés sur la figure 11 mettent en évidence la croissance des bactéries *Escherichia coli* et la présence de colonies directement utilisables (CDU) bien individualisées.

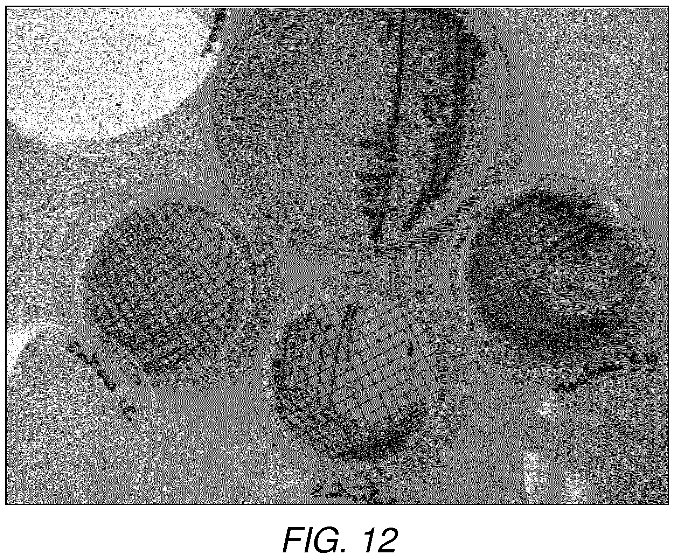

La figure 12 montre des résultats similaires en présence de bactéries *Enterobacter cloacae.*

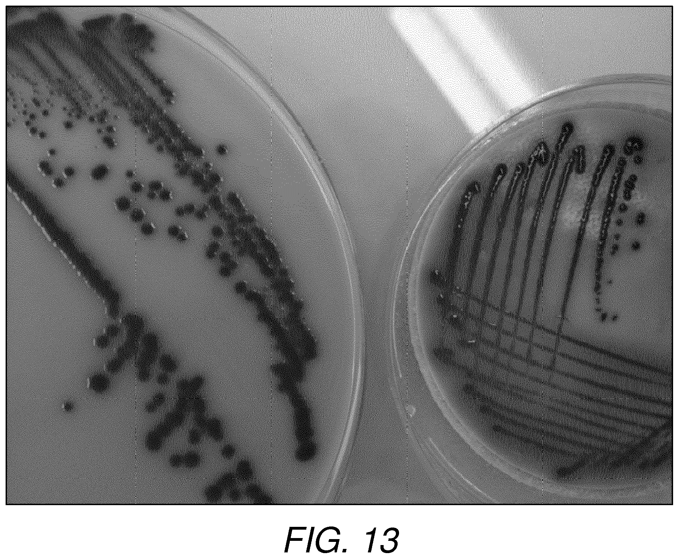

La figure 13 montre des résultats similaires à ceux des figures 11 et 12, en présence d'un milieu gélosé de type UriSelect™ 4.

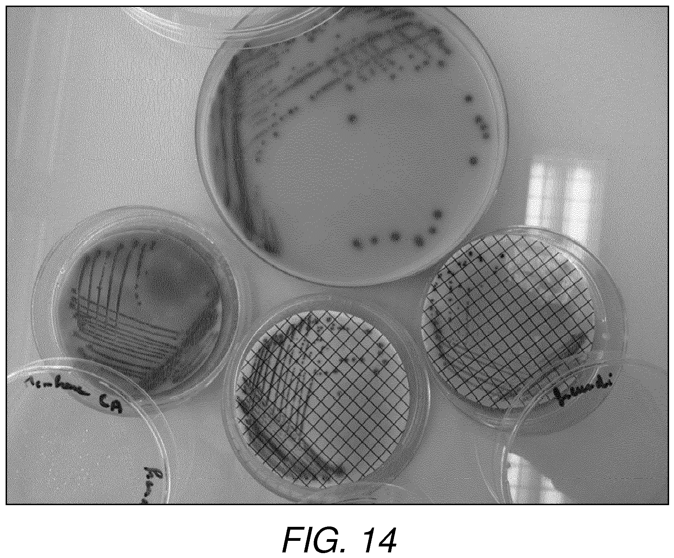

Des résultats similaires sont également visibles sur les contenus des boîtes de Petri montrés en figure 14 en présence d'un milieu de culture UriSelect™ 4 imprégné à sec dans un support non tissé de type Glatfelter, Airlaid 150g/ m² en présence d'une membrane filtrante en acétate de cellulose.

### 3.3.3 Imprégnation avec un milieu de culture de type Chrom ID CPS ID3 déshydraté

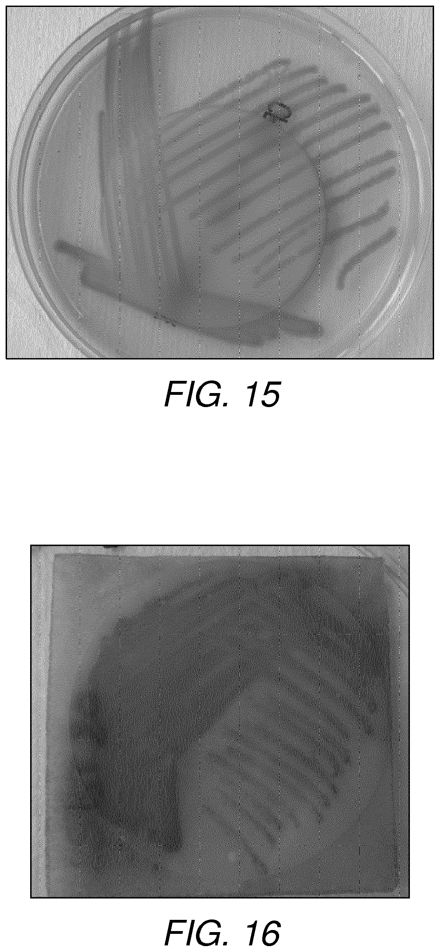

La présente expérimentation concerne un échantillon d'*Enterococcus faecalis* en présence d'un milieu de culture gélosé Chrom ID CPS ID3 (cf figure 15) et d'un milieu déshydraté Chrom ID CPS3 sans agar imprégné dans le support non tissé en présence d'une membrane filtrante en polyester (cf. figure 16).

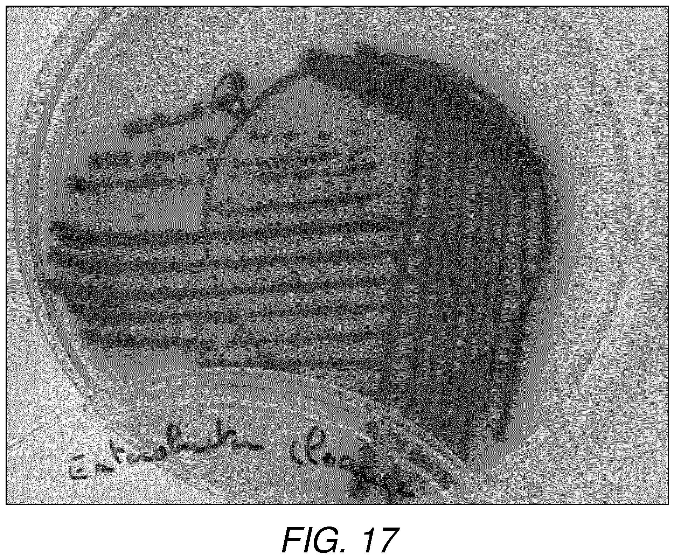

Une autre expérimentation concerne un échantillon d'*Enterobacter cloacae* en présence d'un milieu de culture gélosé de type Chrom ID CPS ID3 et d'un milieu de culture imprégné de type Chrom ID CPS3 déshydraté et sans agar en présence d'une membrane filtrante en polyester (cf. figure 17). Sur cette figure 17, l'on note la présence de colonies directement utilisables (CDU) bien individualisées.

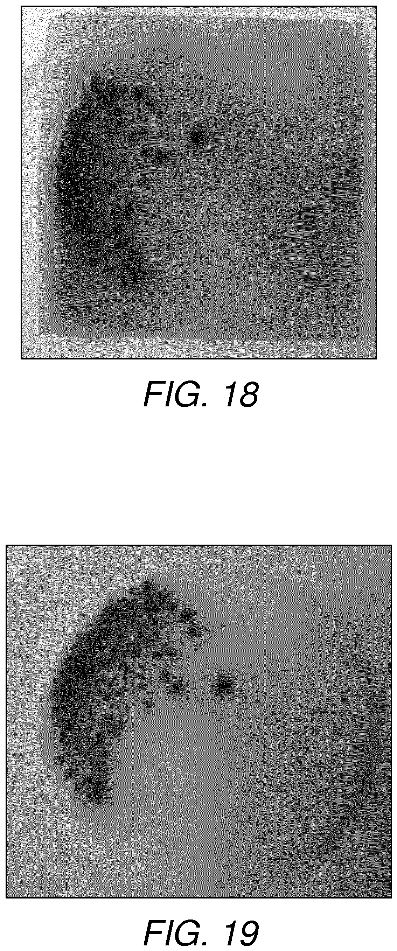

Les figures 18 et 19 montrent les résultats d'une expérimentation mettant en présence un milieu de culture Chrom ID CPS ID3 sans agar imprégné sur un support non tissé Airlaid MH 100 137 en présence d'une membrane filtrante en polyester.

Comme montré sur les figures 18 et 19, l'on note la présence de colonies directement utilisables (CDU) bien individualisées. Alors que l'isolement a été effectué sur membranes filtrantes positionnées sur des milieux de culture déshydraté - et non sur des milieux gélosés - cela n'a pas affecté la croissance bactérienne, laquelle s'est avérée optimale. D'autre part, les morphotypes des colonies obtenues sont similaires à ceux obtenus avec isolement sur membranes poreuses et/ou filtrantes positionnées sur milieu gélosé.

### 3.4 Conclusion

Les résultats des expérimentations relatives à l'exemple 3 indiquent qu'il est possible de réaliser des isolements de micro-organismes sur une couche d'isolement de type membrane filtrante. Notons que ces membranes filtrantes ne sont pas utilisées pour l'action de filtration de liquide, ce qui est leur vocation première, mais pour réaliser l'isolement d'un échantillon pouvant être extrêmement chargé en micro-organismes, ce qui exige d'elles les mêmes qualités de surface que celles que l'on obtient sur les milieux gélosés. D'autre part, l'isolement n'a pas généré de déformations de la membrane filtrante, cette dernière étant restée comme collée à la couche nutritive (support nutritif) sous-jacente sans qu'aucun lien physique ou chimique entre la membrane filtrante et la couche nutritive ne soit nécessaire. Cette intimité de la membrane filtrante avec la couche nutritive après isolement est vérifiée lorsque l'on examine l'intégrité et la continuité du trait de l'isolement au travers de l'agencement des colonies bactériennes.

Outre la compatibilité des membranes poreuses et/ou filtrantes avec l'acte d'isolement microbien, la Demanderesse a démontré que la superposition de la couche d'isolement de type membrane filtrante et de la couche nutritive de type support non tissé imprégné du milieu de culture déshydraté permet la croissance optimale des micro-organismes sur la couche d'isolement comme en témoignent les morphotypes des colonies bactériennes obtenus.

Il apparaît également que le support non tissé imprégné d'un milieu de culture déshydraté représente une alternative valable à la mise en culture des micro-organismes en présence d'un milieu de culture gélosé contenant de l'agar. En effet, la couche nutritive permet des échanges nutritionnels avec les micro-organismes localisés sur la couche d'isolement afin de permettre une croissance microbienne de qualité. Ainsi, la présence d'une couche d'isolement disposée au-dessus d'une couche nutritive imprégnée d'un milieu de culture déshydraté permet d'obtenir des colonies isolées de micro-organismes à la surface de la couche d'isolement. La porosité de la membrane de filtration permet la rétention des micro-organismes à sa surface et le transfert des nutriments solubilisés présents dans le support nutritif à la surface de la membrane de filtration. L'exemple 3 démontre bien que de tels transferts sont optimaux car aucun retard de croissance suggéré notamment par une taille diminuée des colonies n'a été observé. Notons encore une fois que ce transfert est optimal en l'absence de moyen de liaison ou liant entre membrane de filtration et couche nutritive.

D'une manière générale, les échanges en nutriments et en eau entre la couche nutritive et la membrane filtrante permettent une croissance microbienne optimale. Le milieu de culture imprégné est réhydratable ou peut être réhydraté peu de temps avant ou simultanément à l'isolement microbien.

L'exemple 3 démontre notamment que le dispositif objet de l'invention est compatible avec l'isolement et la croissance microbienne.

## Revendications

1. Procédé d'isolement d'au moins un micro-organisme issu d'un échantillon susceptible d'être contaminé par ledit micro-organisme comprenant les étapes suivantes :
(a) fournir un dispositif pour l'isolement de micro-organismes comprenant
∘ une couche inférieure imperméable à l'eau
∘ une couche nutritive, disposée sur la couche inférieure, comprenant un support contenant un milieu de culture déshydraté, ladite couche nutritive étant obtenue par imprégnation, de préférence à sec, dudit support par le milieu de culture déshydraté, puis calandrage
∘ une couche d'isolement perméable aux éléments compris dans la couche nutritive, apte à retenir les bactéries à sa surface et recouvrant tout ou partie de la couche nutritive
∘ une couche supérieure protectrice
(b) déposer un volume déterminé de l'échantillon sur la couche d'isolement
(c) isoler les micro-organismes par épuisement ou par nappage de l'échantillon à l'aide un moyen d'isolement
(d) incuber le dispositif pendant un temps et à une température prédéterminés permettant la croissance des micro-organismes
ledit procédé comprenant également au moins une étape de réhydratation du milieu de culture avec un volume de liquide prédéterminé avant ou simultanément à l'étape b) et/ou c) et/ou d), de préférence avant ou simultanément à l'étape b) et/ou c).

2. Procédé selon la revendication 1, dans lequel la couche d'isolement possède des motifs hydrophiles et/ou hydrophobes.

3. Procédé selon la revendication 1 ou 2, dans lequel le dispositif comprend également un code d'identification tels que les code-barres ou les étiquettes RFID.

4. Procédé selon l'une des revendications 1 à 3, dans lequel le liquide utilisé pour réhydrater le milieu de culture est une solution aqueuse et/ou un liquide contenant des microparticules avec des enveloppes lipidiques.

5. Procédé selon l'une des revendications 1 à 4, dans lequel au moins la couche nutritive et la couche d'isolement sont non liées entre elles au sein dudit dispositif.

6. Procédé selon l'une des revendications 1 à 5, dans lequel ledit support est un support non tissé.

7. Dispositif pour la culture de micro-organismes comprenant :
∘ une couche inférieure imperméable à l'eau
∘ une couche nutritive, disposée sur la couche inférieure, comprenant un support contenant un milieu de culture déshydraté, ladite couche nutritive étant obtenue par imprégnation, de préférence à sec, dudit support par le milieu de culture déshydraté, puis calandrage
∘ une couche d'isolement perméable aux éléments compris dans la couche nutritive, apte à retenir les bactéries à sa surface et recouvrant tout ou partie de la couche nutritive
∘ une couche supérieure protectrice,
dans lequel au moins la couche nutritive et la couche d'isolement sont mécaniquement indépendantes l'une de l'autre.

8. Dispositif selon la revendication 7, ledit dispositif comprenant au moins un réservoir intégré au dispositif et/ou des canaux permettant la réhydratation de la couche nutritive.

9. Dispositif selon la revendication 7 ou 8, dans lequel la couche d'isolement est une membrane poreuse, de préférence une membrane de microfiltration d'un liquide.

10. Dispositif selon l'une des revendications 7 à 9, dans lequel la couche d'isolement contient des motifs hydrophobes et/ou hydrophiles.

11. Dispositif selon l'une des revendications 7 à 10, ledit support est un support non tissé.

12. Utilisation d'un dispositif selon l'une des revendications 7 à 11 pour isoler au moins un micro-organisme.

## Patentansprüche

1. Verfahren zum Isolieren von mindestens einem Mikroorganismus aus einer Probe, die von dem Mikroorganismus kontaminiert sein kann, umfassend die folgenden Schritte:
(a) Bereitstellen einer Vorrichtung zum Isolieren von Mikroorganismen, umfassend
∘ eine untere wasserundurchlässige Schicht
∘ eine Nährschicht, die sich auf der unteren Schicht befindet, umfassend einen Träger, der ein entwässertes Kulturmedium enthält, wobei die Nährschicht durch Imprägnieren, vorzugsweise im trockenen Zustand, des Trägers mit dem entwässerten Kulturmedium erhalten wurde, anschließendes Kalandrieren
∘ eine Isolierschicht, die für in der Nährschicht befindliche Elemente durchlässig ist und die sich dazu eignet, die Bakterien auf ihrer Oberfläche zurückzuhalten und die die Nährschicht ganz oder teilweise abdeckt
∘ eine obere Schutzschicht
(b) Aufgeben eines bestimmten Probevolumens auf die Isolierschicht
(c) Isolieren der Mikroorganismen durch Abreicherung oder durch Belegen der Probe mit Hilfe eines Isoliermittels
(d) Inkubieren der Vorrichtung für einen vorbestimmten Zeitraum und bei einer vorbestimmten Temperatur, um das Wachstum der Mikroorganismen zu gestatten,
wobei das Verfahren auch mindestens einen Schritt der Rehydrierung des Kulturmediums mit einem vorbestimmten Flüssigkeitsvolumen vor oder gleichzeitig mit Schritt b) und/oder c) und/oder d), vorzugsweise vor oder gleichzeitig mit Schritt b) und/oder c), umfasst.

2. Verfahren nach Anspruch 1, wobei die Isolierschicht hydrophile und/oder hydrophobe Einheiten aufweist.

3. Verfahren nach Anspruch 1 oder 2, wobei die Vorrichtung auch einen Identifikationscode wie Strichcodes oder RFID-Etiketten umfasst.

4. Verfahren nach einem der Ansprüche 1 bis 3, wobei es sich bei der zum Rehydratisieren des Kulturmediums verwendeten Flüssigkeit um eine wässrige Lösung und/oder eine Flüssigkeit, enthaltend Mikropartikel mit Lipidhüllen, handelt.

5. Verfahren nach einem der Ansprüche 1 bis 4, wobei zumindest die Nährschicht und die Isolierschicht nicht miteinander in der Vorrichtung verbunden sind.

6. Verfahren nach einem der Ansprüche 1 bis 5, wobei es sich bei dem Träger um einen Vliesträger handelt.

7. Vorrichtung für die Kultur von Mikroorganismen, umfassend:
∘ eine untere wasserundurchlässige Schicht
∘ eine Nährschicht, die sich auf der unteren Schicht befindet, umfassend einen Träger, der ein entwässertes Kulturmedium enthält, wobei die Nährschicht durch Imprägnieren, vorzugsweise im trockenen Zustand, des Trägers mit dem entwässerten Kulturmedium erhalten wurde, anschließendes Kalandrieren
∘ eine Isolierschicht, die für in der Nährschicht befindliche Elemente durchlässig ist und die sich dazu eignet, die Bakterien auf ihrer Oberfläche zurückzuhalten und die die Nährschicht ganz oder teilweise abdeckt
∘ eine obere Schutzschicht,
worin zumindest die Nährschicht und die Isolierschicht voneinander mechanisch unabhängig sind.

8. Vorrichtung nach Anspruch 7, wobei die Vorrichtung mindestens ein in die Vorrichtung integriertes Vorratsbehältnis und/oder Kanäle, wodurch die Nährschicht rehydriert werden kann, umfasst.

9. Vorrichtung nach Anspruch 7 oder 8, wobei es sich bei der Isolierschicht um eine poröse Membran, vorzugsweise um eine Membran zum Mikrofiltrieren einer Flüssigkeit, handelt.

10. Vorrichtung nach Anspruch 7 bis 9, wobei die Isolierschicht hydrophobe und/oder hydrophile Einheiten enthält.

11. Vorrichtung nach einem der Ansprüche 7 bis 10, wobei es sich bei dem Träger um einen Vliesträger handelt.

12. Verwendung einer Vorrichtung nach einem der Ansprüche 7 bis 11 zum Isolieren von mindestens einem Mikroorganismus.

## Claims

1. A method for isolating at least one microorganism from a sample that may be contaminated with said microorganism, comprising the following steps:
(a) supplying a device for isolation of microorganisms comprising
∘ a bottom layer impermeable to water
∘ a nutrient layer, arranged on the bottom layer, comprising a support containing a dehydrated culture medium, said nutrient layer being obtained by impregnation, preferably dry, of said support with the dehydrated culture medium, then calendaring;
∘ an isolating layer permeable to the elements comprised in the nutrient layer, able to retain the bacteria on its surface and covering the whole or a portion of the nutrient layer
∘ a protective top layer
(b) depositing a defined volume of the sample on the isolating layer
(c) isolating the microorganisms by exhaustion or by coating the sample using isolating means
(d) incubating the device for a predetermined time and at a predetermined temperature allowing growth of the microorganisms
said method also comprising at least one step of rehydration of the culture medium with a predetermined volume of liquid before or simultaneously with step b) and/or c) and/or d), preferably before or simultaneously with step b) and/or c).

2. The method as claimed in claim 1, wherein the isolating layer has hydrophilic and/or hydrophobic units.

3. The method as claimed in claim 1 or 2, wherein the device also comprises an identification code such as barcodes or RFID tags.

4. The method as claimed in one of claims 1 to 3, wherein the liquid used for rehydrating the culture medium is an aqueous solution and/or a liquid containing microparticles with lipid envelopes.

5. The method as claimed in one of claims 1 to 4, wherein at least the nutrient layer and the isolating layer are not joined together within said device.

6. The method as claimed in one of claims 1 to 5, wherein said support is a nonwoven support.

7. A device for culture of microorganisms comprising:
∘ a bottom layer impermeable to water
∘ a nutrient layer, arranged on the bottom layer, comprising a support containing a dehydrated culture medium, said nutrient layer being obtained by impregnation, preferably dry, of said support with the dehydrated culture medium, then calendering;
∘ an isolating layer permeable to the elements comprised in the nutrient layer, able to retain the bacteria on its surface and covering the whole or a portion of the nutrient layer
∘ a protective top layer,
in which at least the nutrient layer and the isolating layer are mechanically independent of one another.

8. The device as claimed in claim 7, said device comprising at least one reservoir integrated with the device and/or channels allowing rehydration of the nutrient layer.

9. The device as claimed in claim 7 or 8, wherein the isolating layer is a porous membrane, preferably a membrane for microfiltration of a liquid.

10. The device as claimed in one of claims 7 to 9, wherein the isolating layer contains hydrophobic and/or hydrophilic units.

11. The device as claimed in one of claims 7 to 10, wherein said support is a nonwoven support.

12. The use of a device as claimed in one of claims 7 to 11 for isolating at least one microorganism.
